# EUROPEAN PATENT APPLICATION

(11) **EP 2 676 599 A1**
(43) Date of publication of application: **25.12.2013**
(21) Application number: 13172661.4
(22) Date of filing: 19.06.2013
(51) Int. Cl.: A61B 5/02, A61B 5/0402, A61B 5/0404, A61B 5/021, A61B 5/00, A61B 5/0408, A61B 5/0432

(54) **Cardiovascular monitoring device**

(30) Priority: 21.06.2012 US 201213528871
(71) Applicant: Chou, Chang-An, 8706 Meilen (CH)
(72) Inventor: Chou, Chang-An, 8706 Meilen (CH)

(57) **Abstract**

The present invention relates a cardiovascular monitoring device including a first housing and a second housing, a circuitry with at least a processor, distributed in the first and second housings to form a first unit and a second unit, for providing functions of blood pressure and electrocardiogram measurements, an inflatable cuff connected with the first housing for being used during blood pressure measurement, and at least two electrodes, at least one of which is a dry electrode and mounted on the second housing for contacting the user's hand. The processor can detect an activation of the functions of blood pressure and electrocardiogram measurements, and based on a detecting result, trigger the device to enter different operation modes. The processor can further provide diastolic and systolic blood pressures when the blood pressure measurement function is activated, and provide a heart rhythm information when the electrocardiogram measurement function is activated.

## Description

### FIELD OF THE INVENTION

The present invention relates to a cardiovascular monitoring device with both functions of blood pressure and electrocardiogram measurements, and more particularly to a cardiovascular monitoring device which can provide different operation modes based on the user's requirement.

### BACKGROUND OF THE INVENTION

Modem people pay more and more attention on their cardiovascular health. Blood pressure monitor is one of the most common home-use devices for daily monitoring of cardiovascular health. Not only it is convenient to use, but also the high blood pressure is one of the risk factors related to many kinds of chronic diseases, such as, heart diseases, and diabetes.

Recently, owing to users' demands and also the technical development, the function of the blood pressure monitor has been improved a lot. In addition to the blood pressure, the blood pressure monitor now can provide more information related to the cardiovascular system, e.g., the average heart rate and the indication of irregular heartbeat. However, the function of irregular heart rate detection provided by the blood pressure monitor should only be a referential indication for heart rhythm because it is provided based on the arterial pulses detected.

Arrhythmia is one common kind of heart rhythm problem whose symptom is the rate or rhythm of the heart rate is too fast, too slow, or with an irregular rhythm. Some arrhythmias are life-threatening medical emergencies that can result in cardiac arrest. Others cause symptoms such as an abnormal awareness of heart beat (palpitations), and may be merely uncomfortable. Except of congenital and some heart diseases, some medical conditions such as diabetes and high blood pressure, and stress, caffeine, smoking, alcohol etc. can also affect the heart's natural beating pattern and lead to arrhythmia. Thus, arrhythmia really is a symptom that related to not only heart itself but also the cardiovascular system and physical condition, and should pay more attention to. Up to now, the most accurate way to diagnose arrhythmia is ECG test.

According to the descriptions above, in addition to the blood pressure, it will be better to also consider the electrocardiogram as discussing the cardiovascular health. Therefore, if there can be a device simultaneously provide these two functions, blood pressure measurement and electrocardiogram measurement, it will be a significant improvement in cardiovascular monitoring. This will be very helpful in collecting the two most important data for doctor's diagnosis and also in long-term tracking of cardiovascular health.

Thus, the object of the present invention is to provide a cardiovascular monitoring device which provides the functions of both blood pressure and electrocardiogram measurements.

Another object of the present invention is to provide a cardiovascular monitoring device which provides selectable options for the user to perform the blood pressure and/or the electrocardiogram measurements based on a distributed circuit and structural design.

### SUMMARY OF THE INVENTION

For providing more flexibility and convenience, the cardiovascular monitoring device with the functions of blood pressure and electrocardiogram measurements of the present invention adopts a distributed design which includes a first housing and a second housing, a circuitry with at least a processor, configured to provide functions of blood pressure measurement and electrocardiogram measurement and distributed in the first housing and the second housing to form a first unit and a second unit, an inflatable cuff, connected with the first housing for surrounding a limb of a user during the blood pressure measurement, and at least two electrodes, at least one of which is configured to be a dry electrode and mounted on a surface of the second housing to be contacted by a hand of the user for holding the second housing, wherein the processor is configured to detect an activation of the functions of blood pressure measurement and electrocardiogram measurement, and based on a detecting result, to trigger the device to enter different operation modes, and the processor is further configured to provide a diastolic blood pressure and a systolic blood pressure when the blood pressure measurement function is activated, and to provide a heart rhythm information when the electrocardiogram measurement function is activated.

Preferably, the heart rhythm information can include, but not limited, an average heart rate and/or an indication of arrhythmia, and other information about heart rhythm that can be derived from the acquired electrocardiogram. And, other than the blood pressure and the heart rhythm information, the processor can further configured to provide at least one of ST value, QRS, PR, QTc and parameters related to HRV when the electrocardiogram measurement is activated.

According to the distributed design of the cardiovascular monitoring device of the present invention, the processor(s) can be located in the first unit and/or the second unit, and based on which unit(s) the processor(s) is(are) located in, the arrangement of the circuitry also can be varied. Further, the two units can be implemented to wirelessly communicate or wiredly connected with each other. Therefore, when designing the physical structures and the interaction of the two units of the cardiovascular monitoring device, the present invention can provide the flexibility for conforming to the real demand, and thus, improve the convenience for the user.

The electrical connection between the first and the second units can be achieved by a cable or a pair of connectors, or can be implemented to have a mechanical combination to form an integration. Particularly, the connector can be further configured to perform a data output, e.g., a download process, from one of the unit to an outer device, e.g., a computer. Alternatively, the download process also can be achieved by at least a transmission port, so as to download data from the first unit and/or the second unit.

No matter the two units are wiredly connected or wirelessly communicated with each other, both situations can employ the concept of component sharing to save the cost and also the physical sizes of the units, for example, in the case of wireless communication, two units can share, e.g., the display element and the operation interface, and in the case of wired connection, more components can be shared, such as, filter, amplifier and power source. Accordingly, it can be implemented that the acquired signals are stored in one unit first, in a built-in memory or a removable memory, and then transmitted to the other unit, wiredly or wirelessly, for a further process, such as, calculation, analysis and/or display, without limitation.

In another aspect of the present invention, additionally, the cardiovascular monitoring of the present invention also can further include an external unit configured to communicate with the first and the second units, so that in this case, the external device can be configured to provide options for the user to activate the functions of blood pressure measurement and electrocardiogram measurement, and based on a selected result, to trigger the device to enter different operation modes. Here, the external unit can be implemented to wirelessly communicate or wiredly connect with both units or with one of the units which then wirelessly communicates or wiredly connects with the other unit, without limitation. Furthermore, in addition to providing the interface for the user to access/control the activations of blood pressure and electrocardiogram measurements, the external unit also can be implemented to perform the synchronization with the first and the second units and to provide more algorithms for further calculation and analysis. Therefore, the external unit provides a possibility to introduce extra function(s) into the cardiovascular monitoring device. The external unit can be, for example, but not limited, a smart phone, a PDA, a notebook, or a tablet computer.

The two-unit design of the present invention provides the possibility of performing individual measurement in an independent way. In a preferred embodiment, the separated unit with electrodes mounted thereon provides an independent housing for the user to hold when performing the electrocardiogram measurement. In another preferred embodiment, the wireless design or a detachable electrical connection provides a portable unit, instead of the whole device, for user to carry around. In still another preferred embodiment, the distributed arrangement of circuitry allows the user to select and active the needed function based on the real situation.

Accordingly, the cardiovascular monitoring device of the present invention provides a different distributed design from the prior arts which increases the flexibility as embodying the device and improves the operation convenience for the user. Moreover, through the distributed design with the concept of component sharing, not only the cost can be reduced, but the physical size(s) of the unit(s) also can be reduced to be more suitable for portable and handheld use.

### BRIEF DESCRIPTION OF THE DRAWINGS

A more detailed understanding of the invention may be had from the following descriptions of preferred embodiments, given by way of example, and to be understood in conjunction with the accompanying drawings, wherein:
Fig. 1 shows a circuit diagram of a cardiovascular monitoring device with functions of blood pressure measurement and electrocardiogram measurement according to the present invention;
Fig. 2 is a schematic view of the cardiovascular monitoring device in a first preferred embodiment of the present invention;
Fig. 3 is a schematic view of the cardiovascular monitoring device in a second preferred embodiment of the present invention;
Fig. 4 is a schematic view of the cardiovascular monitoring device in a third preferred embodiment of the present invention;
Fig. 5 is a schematic view of the cardiovascular monitoring device in a fourth preferred embodiment of the present invention;
Fig. 6 is a schematic view of the cardiovascular monitoring device in a fifth preferred embodiment of the present invention; and
Fig. 7 is a schematic view of the cardiovascular monitoring device in a sixth preferred embodiment of the present invention.

### DESCRIPTION OF THE PREFERRED EMBODIMENTS

The present invention is related to a cardiovascular monitoring device which provides, in addition to the functions of measuring blood pressure and electrocardiogram, also a changeable operation mode and a physically separated structure design. Therefore, the user can self-decide that the operation is performed with only the blood pressure measurement, only the electrocardiogram measurement, or both the blood pressure and the electrocardiogram measurements. And, through the distribution of the circuitry into multiple units, the user can selectively use part of the device which conforms to the real demand instead of the whole device, so as to reduce the complexity and also the physical size during operation.

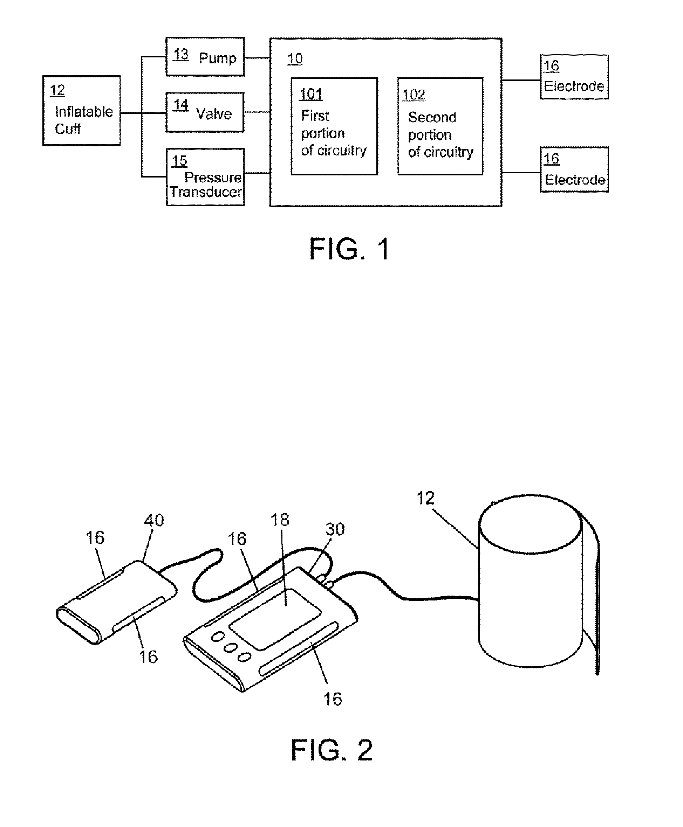

Please refer to Fig. 1, which shows the circuit diagram of a cardiovascular monitoring device with functions of blood pressure measurement and electrocardiogram measurement according to the present invention. As shown, the cardiogram device includes a circuitry 10, an inflatable cuff 12, a pump 13, a valve 14, a pressure transducer 15, and at least two electrodes 16, wherein the circuitry 10 includes the circuits for measuring blood pressure and electrocardiogram, and is divided into two portions for respectively accommodating in two housings so as to form two units. The first portion of circuitry 101 is accommodated in a first housing 30 and the second portion of circuitry 102 is accommodated in a second housing 40. It should be noted that there is no limitation to the division of the circuitry, and it can be varied depending on different demands. For example, in the first portion, the circuitry for detecting blood pressure can be included, and the circuitry for measuring electrocardiogram can be included in the second portion, or the circuitry is distributed without particular purpose, or the circuitry is specialized for division according to the function designed for each unit.

Furthermore, since the circuitry is used to perform the blood pressure measurement and the electrocardiogram measurement, the basic circuitry therefor should be included therein, such as, a processor and preloaded program for controlling the measurements, at least an A/D converter(s) for digitizing the received signals, and other common-used electrical components, e.g., a filter, an amplifier, a display element, and a power source. Since these are common for one skilled in the art, the detailed descriptions are omitted.

Moreover, the device can further includes a memory in the first unit and/or the second unit, depending on the design, for the storage of signals, analysis results and/or related information, and more preferably, the memory can be implemented to be removable. For outputting the stored data, the removable memory is a convenient choice, for example, the removable memory of one unit can be accessed by the other unit, and/or the user can bring the removable memory with the measurement/analysis results stored therein to the doctor. In addition to using the removable memory, the stored data also can be outputted to an outer device (e.g., a computer) or outputted to the other unit through a wired connection (e.g., via at least a transmission port) or a wireless communication.

It also should be noted that, the communication between the first unit and the second unit can be implemented as wired or wireless. For example, two units can communicate via a wired electrical connection, for example, a connection cable or detachable connectors, or the two units can communicate wirelessly through further employing the wireless modules. The implementation can be varied without limitation.

In the present invention, the measurement of electrocardiogram is achieved by contacting the electrodes with different portions of user's skin. The most convenient way for the user is to implement the electrode as dry electrode mounted on the surface of the device. Here, the surface for mounting the dry electrode(s) can be any portion of the device that can be contacted by the user, e.g., the surfaces of the housings 30, 40 and the surfaces of the cuff 12, so as to provide the user an easy way to achieve this non-invasive electrocardiogram measurement, such as, by holding, pressing, or rejecting against the housing or the contacting surface. When the dry electrode is employed, for achieving the electrocardiogram measurement, the user can directly contact the electrode without the medium, e.g., the conductive gel, so the measurement can be performed at any time. And, because the dry electrode is uneasily destroyed and can be easily maintained, the inconvenience of replacing the electrode is also avoided. Here, it can be both or only one of the electrodes implemented as the dry electrode(s) without limitation.

In a preferred embodiment, both electrodes are implemented as dry electrodes, and according to the positions of the electrodes, the operation manner can be varied. For example, the user can use one hand to hold the housing for contacting one electrode on the surface thereof and simultaneously moving the housing to contact the other electrode with another portion of body surface, such as, another limb, e.g., the other hand or a leg, or the chest. Or, the user can use both hands to contact two electrodes on one housing or respectively on two housings. Or, the user can use one hand to contact one electrode on the housing and the other to contact the other electrode on the inflatable cuff. In another preferred embodiment, it also can be only one electrode implemented as the dry electrode, and the other implemented as another kind of electrode, such as, a patch electrode or a cup electrode. In this case, for example, the user can attach the patch electrode to the chest and contact the dry electrode by one hand. Thus, there is no limitation.

In addition, the number and function of the electrodes 16 are also unlimited. For example, it can have more than two electrodes to obtain more information about the heart, or it can be designed that the user can self-decide the usage of the electrodes, e.g., when there has the electrode mounted on the cuff, the user can select to use this particular electrode only as the blood pressure measurement is performed.

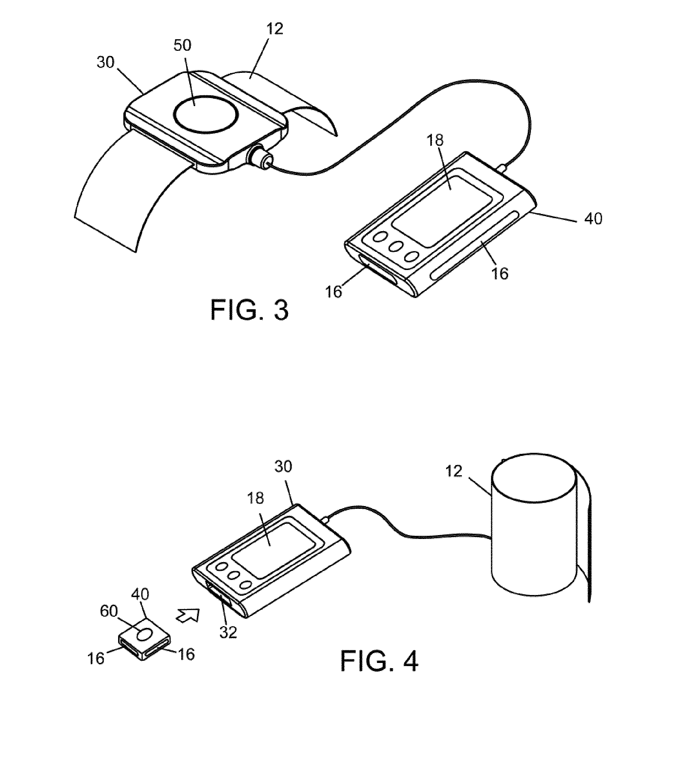

The inflatable cuff 12 required for detecting the blood pressure can be implemented to, for example, combine with the first housing 30 and/or the second housing 40 (as shown in Fig. 3, Fig. 5 and Fig. 7), or connect with the unit via a connection cable (as shown in Fig. 2 and Fig. 4). Just like the blood pressure monitors that can be seen in the market, the cuff can surround the wrist, the arm or other position during operation.

The cardiovascular monitoring device of the present invention also can include a display element 18 for showing the results and related information and an operation interface for inputting. Here, the display element and the operation interface can be arranged to conform to different demands, for example, the display element and/or the operation interface can be located on the first unit only, the second unit only, or both the units, without limitation. Alternatively, the display element and/or the operation interface can be separated to locate on an independent unit, e.g., an external unit, electrically connected to or wirelessly communicate with the first and/or the second unit.

In the present invention, it is important that the components in different units can be shared, for example, the display element, the operation interface, or some basic circuits, so as to reduce the cost and also the physical size. And, the reduced physical size is particular benefit for the holding operation for electrocardiogram measurement.

Then, because in the present invention two units are employed, the circuitry distributed in the two units can include one or more processors, for example, only one processor in one of the units, two processors respectively in two units, or even, more than one processor in one unit. And, when each unit is implemented to have the processor, the two units can have a communication therebetween. Therefore, there is no limitation for the distribution of the circuitry and also the number and type of components included in the circuitry.

Owing to the two-unit design of the present invention, when operating, the user can select the function based on the real demands at the time to activate the blood pressure measurement only, activate the electrocardiogram measurement only, or to activate both. And, since the circuitry is distributed in two housings, the usage of every component should depend on which function is activated and not which housing it is located.

Depending on which function(s) is(are) activated, independently or simultaneously, the device will accordingly enter a different operation mode. For example, if only the function of blood pressure measurement is activated, by entering an operation mode of blood pressure measurement, the processor will utilize the needed components to perform the blood pressure measurement, no matter which unit the components are located in. And, similarly, as the function of electrocardiogram measurement is activated independently, the device enters an operation mode of electrocardiogram measurement and the processor will only utilize the components related thereto. Then, if both functions are activated, the device enters an operation mode of performing both measurements.

Here, owing to the separability, other than the operation modes described above, the device also can provide other selections. For example, an operation mode can be both units are turned on without performing measurement but the processor acquires data from a memory located in another unit. Alternatively, another operation mode can be at least one unit is turned on for transmitting the data to an outer device, such as, a computer. Therefore, the user can freely select the suitable operation mode without limitation.

As described above, for providing the analysis result, the device will have a preloaded program including several algorithms corresponding to the signals that can be acquired. Here, because the device according to the present invention can measure both the blood pressure and the electrocardiogram, there can have more analyzing selections than the blood pressure monitor alone or the electrocardiograph alone. The algorithms can be divided into three groups. One is related to blood pressure measurement, for example, but not limited, diastolic blood pressure, systolic blood pressure and heart rate. One is related to electrocardiogram measurement, for example, but not limited, heart rate, arrhythmia, ST value, QRS, PR, QTc, and parameters related to HRV (Heart Rate Variability) including time domain statistical parameters, e.g., SDNN, RMSSD, NN50, and pNN50, time domain geometrical parameters, e.g., RR triangular index, and TINN, and frequency domain parameters, e.g., LF, HF, and LF/HF. The other is related to both, for example, but not limited, artifact removal and PTT (pulse transit time, which is the time it takes the pulse pressure waveform to propagate through a length of the arterial tree).

The most difference from the conventional blood pressure monitor is the device of the present invention can provide the information that must come from the electrocardiogram, such as the information about heart rhythm, e.g., the average heart rate, and the indication of arrhythmia, and other information judged from the waveform of electrocardiogram, e.g., ST values which are related to myocardial infarction.

Therefore, the user not only can obtain more kinds of analysis results but also in some level can have more accurate results, e.g., comparing the indication of irregular heartbeat with the indication of arrhythmia. Besides, because the blood pressure and the electrocardiogram are actually relative to each other, two kinds of signals can have a cross reference for obtaining the information representing other physiological conditions, such as, pulse transit time. Further, the comparison between the arterial pulses and ECGs also is helpful to remove the artifact.

Corresponding to different operation modes, the user can have different options of algorithms for obtaining different analysis/calculation results or of actions for the following procedure. For example, when the blood pressure measurement is performed, the possible algorithms are diastolic blood pressure, systolic blood pressure and heart rate, when the electrocardiogram measurement is performed, the algorithms for provision of arrhythmia, heart rate, ST value, QRS, PR, QTc, and parameters related to HRV can be executed, and when both measurements are performed, all algorithms described above plus PTT can be executed. Alternatively, when it has decided to perform the electrocardiogram measurement, the user can further select to use the electrode(s) whose position(s) is(are) more convenient to contact under the current condition, such as, to use the electrode(s) on the housing and not use the electrode(s) on the cuff. Alternatively, as described above, the user also can select not to perform the measurement but to, for example, acquire the stored data from another unit or output the data to a computer. There is no limitation.

Here, it should be noted that the description of both measurements are performed means the two measurements are activated within a particular time period. That is, two measurements do not need to be started at the same time. For the present invention, the time period can be ranged from 0 to 10 minutes, and also, the two measurements can be overlapped or not. This time period is employed for providing more reasonable average physiological conditions of the user.

Following, more detailed embodiments will be described. However, it should be noted that the embodiments are for illustration only and not for limitation. One skilled in the art can have modifications or variations of the embodiments without departing from the scope of the present invention.

As shown in Fig. 2 to Fig. 7, the first unit and the second unit of the cardiovascular monitoring device according to the present invention can have different arrangements, shapes and connection manners for achieving different usage purposes, circuitry designs and also operation convenience, without limitation.

In the embodiment shown in Fig. 2, the first unit/housing 30 is implemented to perform the blood pressure measurement alone. The surfaces of the first unit 30 have electrodes 16 mounted thereon, so that by electrically connecting to the second unit 40, the electrodes 16 on the first housing 30 and the electrodes 16 on the second unit 40 can work together to achieve the electrocardiogram measurement. This arrangement is particularly suitable for performing the electrocardiogram measurement through both hands simultaneously holding two units. Alternatively, through a selection by the user, the electrocardiogram measurement also can be performed using the electrodes on the second unit only. Alternatively, the electrode(s) 16 on the surface(s) of the first housing 30 also can be implemented to locate on the surface of the cuff 12 (not shown) which is combined with the first housing. The electrode only needs to locate on the surface which is contactable without limitation. The display element 18 on the first unit can be used to show the measurement result and related information.

In another embodiment, as shown in Fig. 3, the second unit/housing 40 is implemented to be able to utilize the electrodes 16 on the surfaces thereof to perform the electrocardiogram measurement alone, e.g., by the user's different skin portions respectively contact different electrodes 16, and to use the display element 18 to show the measurement process/result. And, the first unit/housing 30 is implemented to perform the blood pressure alone, e.g., by pressing the start button 50, and store the results. Then, after being connected with the second unit/housing 40, the results of blood pressure measurement stored in the first unit can be shown by the display element 18 on the second unit. In this embodiment, the advantage of putting all electrodes on the second unit is the user can directly have a separate unit for holding operation which is more convenient and smaller than holding a non-separate device.

Therefore, according to the embodiments above, the present invention provides a new concept for distributing the circuitry for blood pressure and electrocardiogram measurements into two housings. The design of two units not only provides the convenience of holding operation, but also provides the possibility to reduce the physical size and the cost due to the sharing of components.

Preferably, the wired connection between the first and the second units can be implemented to be detachable, e.g., through detachable connectors, so that when the user wants to use the unit which can be operated alone, the other unit can be separated therefrom, as the example shown in Fig. 3, if the second unit becomes detachable from the first unit, the holding operation of the second unit for electrocardiogram measurement may even more convenient. And, owing to the detachment, it will be more preferable to further employ the connection port to achieve a data output, e.g., a download process from the unit to an outer device, e.g., a computer.

In another embodiment, as shown in Fig. 4, the first unit 30 can be combined with the cuff to perform the blood pressure measurement alone. The second unit 40 can have the electrodes 16 mounted on the surfaces for performing the electrocardiogram measurement alone. The electrocardiogram measurement can be started by pressing the power button 60 with the hand holding the housing. Even, one of the electrodes can be directly mounted on the button 60, so that the user can easily contact the electrode when pressing the button to start the measurement. After measurement, the ECG signals/results stored in the second unit can be displayed by the display element 18 of the first unit 30. In the embodiment shown in Fig. 4, the first unit 30 is designed to have an indentation for receiving the second unit 32, that is, the two units are not only electrically connected but also mechanically combined together to form an integration. The type of combination can be achieved by, for example, hooking, sliding or insertion, without limitation.

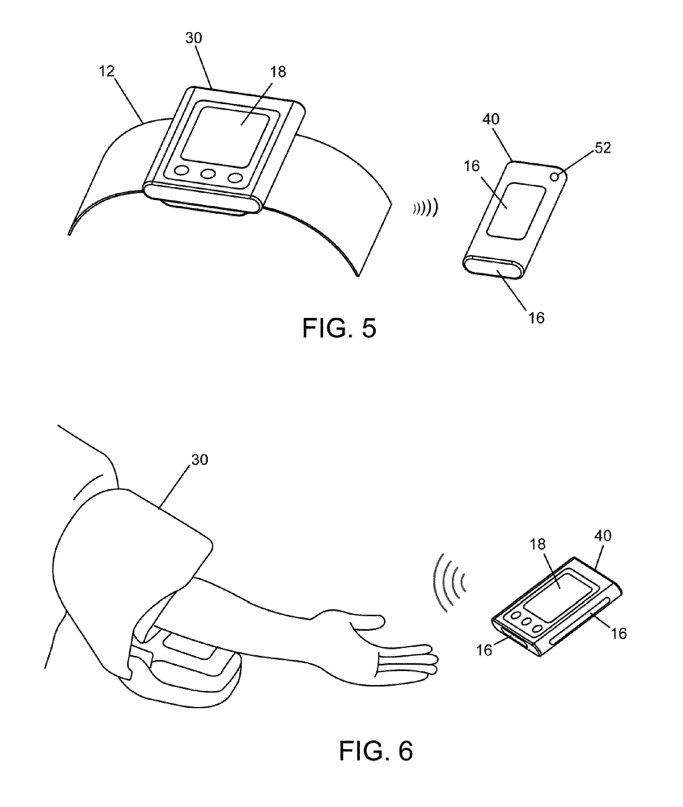

In still another embodiment, as shown in Fig. 5, the first unit/housing 30 (blood pressure measurement) and the second unit/housing 40 (electrocardiogram measurement) are implemented to wirelessly communicate with each other which means both unit include a processor. In this case, the communication between the two units can have many choices. For example, it can be implemented that the first unit controls the operation of the second unit when the communication therebetween is achieved, or the second unit can perform the electrocardiogram measurement alone when there is no communication with the first unit. As to data storage, result analysis and information display, there also can have different possibilities. In one case, the signals acquired by the second unit can be transmitted to the first unit in real time for display, storage and analysis, and additionally, the second unit can further store the signals in the memory thereof for providing a backup since it is possible for the wireless transmission to be instable. In another case, the second unit can store the acquired signals in the memory thereof first, and after the measurement, transmit to the first unit for analysis and display. In still another case, the second unit can perform the analysis of the measurement before the transmission to the first unit, so that first unit can directly receive and show the analysis result. In further another case, the second unit can further include an indicating element 52 for instantly providing the information about measurement for the user, and after the measurement, the more detailed information can be shown by the display element 18 of the first unit. Therefore, it can be varied for conforming to different design concepts without limitation. Through the wireless communication, not only the connection cable can be eliminated, but the measurement results, e.g., blood pressure and heart rate, and/or the ECG trace/waveform also can be shown for the user in real time. In this embodiment, another advantage of employing the wireless communication is the second unit can be used alone to perform the electrocardiogram measurement and since the physical size of the second unit has been reduced due to the component sharing, e.g., the sharing of display element, it will be more suitable for carrying. Thus, this provides the possibility for the user to measure the electrocardiogram at any time and place.

Consequently, in the present invention, it is important that different functions can be activated separately. When the user has the need to use a portion of the device, he or she can take only the needed portion of device to perform the measurement for blood pressure or electrocardiogram rather than taking the whole device for only partial function.

In a particular embodiment, as shown in Fig. 6, the first housing 30 is directly implemented to be positioned on the desk with the cuff included therein. The second unit 40 is implemented to be a handheld ECG device which can wirelessly communicate with the first unit. Here, the blood pressure measurement performed by the first unit is controlled by the handheld second unit, and the results of blood pressure measurement can be transmitted to the second unit for immediate display. The second unit can utilize the electrodes 16 on the surfaces thereof to perform electrocardiogram measurement. Therefore, it will be like to use a remote controller of the blood pressure measurement only that the remote controller is further equipped with the function of electrocardiogram measurement. Particularly, in addition to separately performing the measurements, the user also can directly initiate the blood pressure measurement and the electrocardiogram measurement by pressing the button on the second unit so as to further simplify the operation procedure.

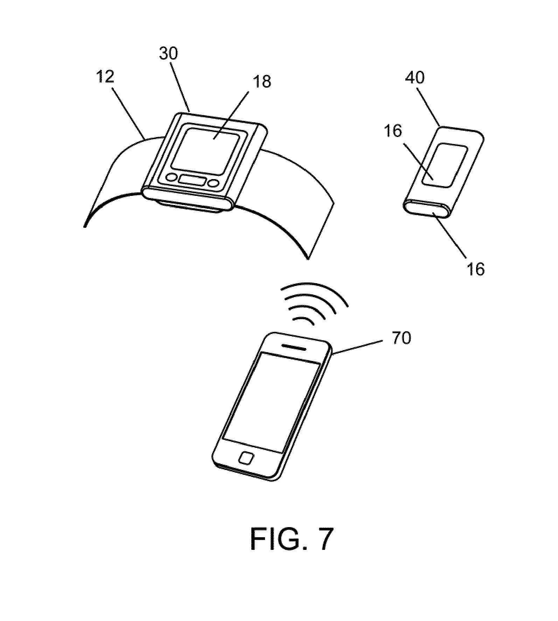

In still another particular embodiment, as shown in Fig. 7, an external unit 70 can be further provided for controlling the operation of both units 30, 40. For example, the external unit 70 can be utilized to control the units, to select the operation mode, to select the algorithm(s) for calculation and/or analysis, and/or to display the measurement procedure and/or result for the user. Moreover, the external unit 70 also can include additional algorithms for further calculation/analysis. It should be noted that the data transmission from the two units to the external unit can be done during the measurement or after the measurement, without limitation. And, the communication between the external unit 70 and the first and the second units 30, 40 can also include a synchronization, so as to further facilitate the operations of cross reference and artifact removal. The external unit can be, for example, but not limited, a smart phone, a PDA, a notebook computer, or a tablet computer which can provide the operation interface for controlling and more preferably a display for showing the information. Here, although Fig. 7 illustrates an example that the external unit wirelessly communicates with the first unit and/or the second unit, the external unit also can be implemented to wiredly connect with the first unit and/or the second unit, without limitation.

Accordingly, in practice, as described above, the design of the first unit and the second unit can have different kinds of variations depending on the real demand and will not be limited.

Moreover, for facilitating the contact-type electrocardiogram measurement, the electrodes, especially the dry electrode(s), (partial or all) can be connected to a sensor for detecting and notifying the user if the contact on the electrode is proper, for example, a pressure detecting element can be used to detect the applied force on the electrode, e.g., a pressure sensor, or a touch sensing element can be used to sense if the electrode is contacted, e.g., through sensing the change of capacitance or resistance. Alternatively, it also can simply employ a switch to sense the force applied on the electrode. Accordingly, it can be further implemented that the electrocardiogram measurement starts automatically when the sensor or the switch senses a proper contacting force or other physical condition change applied on the electrode, such as, reaches a preset value, and even, it can be implemented that the device is initiated thereby.

Besides, the function of the cardiovascular monitoring device according to the present invention also can be expanded through an expansion port. For example, by connecting with an oximeter, a third physiological signals related to the cardiovascular health can be provided, and thus, further information could be obtained by cross reference between multiple signals. Alternatively, it also can be implemented to connect with more electrodes, such as, via a connecting cable, so as to obtain more information about the user's heart. This function expansion can be on the first unit and/or the second unit which depends on the user's selection.

In the aforesaid, the cardiovascular monitoring device with both functions of blood pressure measurement and electrocardiogram measurement according to the present invention, different from the prior arts, adopts a distributed design for the circuitry and structure arrangement so as to maximize the functionality and operation selectivity by providing the user the possibility and the convenience to select the operation mode which mostly conforms to the real need. The provision of multiple operation modes by employing the novel distributed design with the connection between two units can be beneficial to reduce not only the manufacturing cost but also the physical size of the device through sharing the electrical components, and further to provide the information which should be based on multiple physiological signals.

The above examples and disclosures are intended to be illustrative and not exhaustive. These examples and descriptions will suggest many variations and alternatives to one of ordinary skill in this art. All these alternatives and variations are intended to be included within the scope of the attached claims. Those familiar with the art may recognize other equivalents to the specific embodiments described herein which equivalents are also intended to be encompassed by the claims attached hereto.

## Claims

1. A cardiovascular monitoring device, comprising:
a first housing and a second housing;
a circuitry with at least a processor, configured to provide functions of blood pressure measurement and electrocardiogram measurement and distributed in the first housing and the second housing to form a first unit and a second unit;
an inflatable cuff, connected with the first housing for surrounding a limb of a user during the blood pressure measurement; and
at least two electrodes, at least one of which is configured to be a dry electrode and mounted on a surface of the second housing to be contacted by a hand of the user for holding the second housing,
**characterized in that**
the processor is configured to detect an activation of the functions of blood pressure measurement and electrocardiogram measurement, and based on a detecting result, to trigger the device to enter different operation modes, and
the processor is further configured to provide a diastolic blood pressure and a systolic blood pressure when the blood pressure measurement function is activated, and to provide a heart rhythm information when the electrocardiogram measurement function is activated.

2. The device as claimed in claim 1, **characterized in that** the heart rhythm information includes at least one of an average heart rate and an indication of arrhythmia.

3. The device as claimed in claim 1, **characterized in that** the processor is further configured to provide at least one of ST value, QRS, PR, QTc and parameters related to HRV when the electrocardiogram measurement is activated.

4. The device as claimed in claim 1, **characterized in that** the processor is included in the first unit and further configured to perform a data acquisition from the second unit.

5. The device as claimed in claim 1, **characterized in that** the processor is included in the second unit and further configured to perform a data acquisition from the first unit.

6. The device as claimed in claim 1, **characterized in that** the first unit and the second unit are wiredly connected or wirelessly communicated with each other.

7. The device as claimed in claim 6, **characterized in that** the wired connection between the first unit and the second unit is implemented to be detachable via connectors respectively mounted thereon.

8. The device as claimed in claim 7, **characterized in that** the respective connector is further configured to achieve a data output.

9. The device as claimed in claim 1, **characterized in that** a memory and/or a display is further included in the first unit and/or the second unit.

10. The device as claimed in claim 9, **characterized in that** the memory is removable.

11. A cardiovascular monitoring device, comprising:
a first housing and a second housing;
a circuitry, configured to provide functions of blood pressure measurement and electrocardiogram measurement and distributed in the first housing and the second housing to form a first unit and a second unit;
an inflatable cuff, connected with the first housing for surrounding a limb of a user during the blood pressure measurement;
at least two electrodes, at least one of which is configured to be a dry electrode and is mounted on a surface of the second housing to be contacted by a hand of the user for holding the second housing; and
an external unit, configured to communicate with the first and the second units,
wherein
the external device is further configured to provide options for the user to activate the functions of blood pressure measurement and electrocardiogram measurement, and based on a selected result, to trigger the device to enter different operation modes; and
a preloaded program is further provided to provide a diastolic blood pressure and a systolic blood pressure when the blood pressure measurement function is activated, and to provide a heart rhythm information when the electrocardiogram measurement function is activated.

12. The device as claimed in claim 11, **characterized in that** the external unit further comprises algorithms for calculation and analysis.

13. The device as claimed in claim 11, **characterized in that** the external unit is configured to perform a synchronization with the first and the second units.

14. The device as claimed in claim 11, **characterized in that** the external unit is wiredly connected with or wirelessly communicated with the first unit and/or the second unit.
